# EUROPEAN PATENT SPECIFICATION

(11) **EP 4 312 810 B1**
(45) Date of publication and mention of the grant of the patent: **06.05.2026**
(21) Application number: 22712654.7
(22) Date of filing: 18.03.2022
(51) Int. Cl.: A61B 17/064, A61B 17/072, A61B 17/115, A61B 17/00

(54) **SURGICAL STAPLE CARTRIDGE**
KARTUSCHE FÜR CHIRURGISCHE KLAMMERN
CARTOUCHE D'AGRAFES CHIRURGICALES

(30) Priority: 26.03.2021 US 202163166380 P; 10.03.2022 US 202217691424
(43) Date of publication of application: 07.02.2024
(73) Proprietor: Covidien LP, Mansfield, MA 02048 (US)
(72) Inventor: DIAZ-CHIOSA, Olesea, Naugatuck, Connecticut 06770 (US); KNAPP, Robert H., North Haven, Connecticut 06473 (US); ESCHBACH, Matthew S., North Haven, Connecticut 06473 (US); CHOWANIEC, Matthew J., North Haven, Connecticut 06473 (US); MARINELLI, Johana M., North Haven, Connecticut 06473 (US)
(74) Representative: Zimmermann & Partner Patentanwälte mbB
(86) International application number: PCT/IB2022/052485
(87) International publication number: WO 2022/200960

(56) References cited:
- EP-A1- 3 818 949
- EP-A2- 3 954 301
- US-A1- 2004 254 608
- US-A1- 2017 028 184
- US-A1- 2019 046 192

## Description

### CROSS-REFERENCE TO RELATED APPLICATION

This application claims the benefit of and priority to U.S. Provisional Patent Application No. 63/166,380, filed March 26, 2021.

### FIELD

This disclosure is directed a staple cartridge including spaced rows of staples, and more particularly, to a staple cartridge including spaced rows of staples that are adapted to enhance cell migration toward a wound site.

### BACKGROUND

When a surgical stapling and dissecting device is operated to remove tissue from a patient, a knife of the device is advanced to dissect the tissue along a cut line. This creates a wound that is sealed with rows of staples that are ejected from the device and formed through the tissue along the cut line as the tissue is dissected. The staple rows seal the wound by providing pressure to the cut line to maintain hemostasis. However, staple rows fail when the wound does not heal fast enough to prevent the stapled tissue from losing its integrity due to natural degradation.

A continuing need exist in the surgical stapling and dissecting arts for a device that can minimize healing time required for stapled tissue.
US2019/046192A1 describes buttress systems and methods for surgical stapling devices and end effectors.

### SUMMARY

The invention is defined by the appended independent claim. Optional features are set out in the dependent claims.

One aspect of the disclosure is directed to a staple cartridge including a cartridge body and a plurality of staples. The cartridge body defines a plurality of staple pockets and a central knife slot. The staple pockets are arranged in a plurality of rows on each side of the central knife slot. The plurality of rows includes an inner row and an outer row. Each of the plurality of staple pockets receives one of the plurality of staples. Each of the plurality of staples received in the inner rows of staple pockets is formed of a first metal having a first anodic index and each of the plurality of staples received in the outer rows of staple pockets is formed of a second metal having a second anodic index that is different from the first anodic index.

Another aspect of the disclosure is directed to a surgical stapling device including a handle assembly, an elongate body extending distally from the handle assembly, and a staple cartridge including a cartridge body and a plurality of staples. The cartridge body defines a plurality of staple pockets and a central knife slot. The staple pockets are arranged in a plurality of rows on each side of the central knife slot. The plurality of rows includes an inner row and an outer row. Each of the plurality of staple pockets receives one of the plurality of staples. Each of the plurality of staples received in the inner rows of staple pockets is formed of a first metal having a first anodic index and each of the plurality of staples received in the outer rows of staple pockets is formed of a second metal having a second anodic index that is different from the first anodic index.

Yet another aspect of the disclosure is directed to a staple cartridge including a cartridge body and a plurality of staples. The cartridge body defines a plurality of staple pockets and a central knife slot. The staple pockets are positioned on each side of the central knife slot and include first staple pockets positioned along the central knife slot and second staple pockets positioned further from the central knife slot. Each of the plurality of staple pockets receives one of the plurality of staples. Each of the plurality of staples received in the first staple pockets is formed of a first metal having a first anodic index and each of the plurality of staples received in the second staple pockets is formed of a second metal having a second anodic index that is different from the first anodic index.

Another aspect of the disclosure is directed to a staple cartridge including an annular staple cartridge body and a plurality of staples. The annular cartridge body defines a central opening and staple pockets that are arranged to define inner and outer rings positioned about the central opening. Each of the plurality of staple pockets receives one of the plurality of staples. At least some of the plurality of staples received in the inner ring of the staple pockets are formed of a first metal having a first anodic index and at least some of the plurality of staples received in the outer ring of the staple pockets are formed of a second metal having a second anodic index that is different from the first anodic index. The first and second anodic indexes are selected to create a voltaic cell to drive a voltage within tissue to which the plurality of staples are applied.

Yet another aspect of the disclosure is directed to a surgical stapling device including a handle assembly, an elongate body, and a staple cartridge, and an anvil assembly. The elongate body extends distally from the handle assembly and includes an anvil retainer. The staple cartridge includes a cartridge body and a plurality of staples. The cartridge body defines a central opening and staple pockets that are arranged to define inner and outer rings positioned about the central opening. Each of the plurality of staple pockets receives one of the plurality of staples. At least some of the staples received in the inner ring of the staple pockets are formed of a first metal having a first anodic index and at least some of staples received in the outer ring of the staple pockets are formed of a second metal having a second anodic index that is different from the first anodic index. The first and second anodic indexes are selected to create a voltaic cell to drive a voltage within tissue to which the plurality of staples are applied. The anvil assembly is supported on the anvil retainer and is movable in relation to the staple cartridge between advanced and retracted positions to move the stapling device between open and clamped positions.

Another aspect of the disclosure is directed to a staple cartridge that includes a cartridge body and a plurality of staples. The cartridge body defines a central opening and staple pockets positioned about the central opening. The staple pockets including a first ring of staple pockets positioned adjacent the central opening and a second ring of staple pockets positioned further from the central opening. Each of the staple pockets receives one of the plurality of staples. Each of the plurality of staples received in the first ring of the staple pockets is formed of a first metal having a first anodic index and each of the plurality of staples received in the second ring of the staple pockets is formed of a second metal having a second anodic index that is different from the first anodic index. The first and second anodic indexes are selected to create a voltaic cell to drive a voltage within tissue to which the plurality of staples are applied.

In aspects of the disclosure, the first anodic index is less than the second anodic index.

In some aspects of the disclosure, the first metal is magnesium, and the second metal is titanium.

In certain aspects of the disclosure, the first anodic index is about -1.75V and the second anodic index is about -.30V.

In aspects of the disclosure, the plurality of rows on each side of the central knife slot includes a middle row positioned between each of the inner and outer rows.

In some aspects of the disclosure, the staples in the middle row are formed of the second metal.

In certain aspects of the disclosure, the staples in the middle row are formed of the first metal.

In aspects of the disclosure, the staple pockets are arranged to define a middle ring that is positioned between the inner and outer rings, and each of the staple pockets of the middle ring receive one of the staples.

In some aspects of the disclosure, at least some of the staples in the middle ring are formed of the second metal.

In certain aspects of the disclosure, at least some of the staples in the middle ring are formed of the first metal.

In aspects of the disclosure, the stapling device includes a handle assembly, and the elongate body is supported by and extends distally from the handle assembly.

In some aspects of the disclosure, the anvil assembly is releasably coupled to the anvil retainer.

Other features of the disclosure will be appreciated from the following description.

### BRIEF DESCRIPTION OF THE DRAWINGS

Various embodiments of the disclosed staple cartridge are described herein below with reference to the drawings, wherein:
FIG. 1 is a side perspective view of a surgical stapling device including a tool assembly having an exemplary embodiment of the disclosed staple cartridge;
FIG. 2 is a side perspective view of the staple cartridge illustrated in FIG. 1 with staples of the staple cartridge separated from the staple cartridge;
FIG. 3 is a top view of tissue sections dissected and stapled with the staple cartridge illustrated in FIG. 2 with staple rows formed in the tissue;
FIG. 4 is a side perspective view of an alternate version of the surgical stapling device shown in FIG. 1 with an anvil assembly separated from an anvil retainer of the surgical stapling device;
FIG. 5 is a side perspective view of the distal portion of the reload assembly of the stapling device shown in FIG. 4 with staples of the reload assembly shown in phantom;
FIG. 6 is an enlarged view of the indicated area of detail shown in FIG. 5;
FIG. 6A is a view from the distal end of the surgical stapling device shown in FIG. 4 with the anvil assembly removed;
FIG. 7 is a side view of the surgical stapling device shown in FIG. 4 positioned within a body vessel;
FIG. 8 is a cross-sectional view taken along section line 8-8 of FIG. 7; and
FIG. 9 is a cross-sectional view taken along section line 9-9 of FIG. 8.

### DETAILED DESCRIPTION

The disclosed staple cartridge will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure. In addition, directional terms such as front, rear, upper, lower, top, bottom, distal, proximal, and similar terms are used to assist in understanding the description and are not intended to limit the disclosure.

The disclosed staple cartridge is adapted to enhance cell migration towards a wound site when staples are formed in tissue. To enhance cell migration, the rows of staples of the staple cartridge are adapted to create an electrical potential within the tissue in the area of a cut line in tissue. In embodiments, the electrical potential is created within the tissue by applying spaced rows of staples into tissue, wherein the staples in the spaced rows are formed of dissimilar metals having different anodic indexes to create a voltage gradient in the tissue and enhance cell migration toward the cut line.

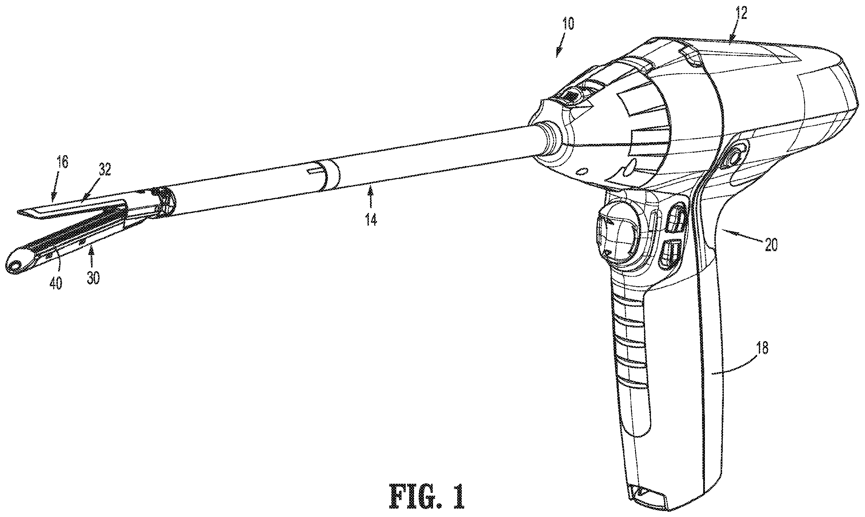

FIG. 1 illustrates a surgical stapling device shown generally as stapling device 10 that includes a handle assembly 12, an elongate body or adapter 14, and a tool assembly 16. In the illustrated embodiment, the handle assembly 12 is powered and includes a stationary handgrip 18 and actuation buttons 20. The actuation buttons 20 are operable to actuate various functions of the tool assembly 16 via the adapter 14 including approximation, stapling, and dissection. In embodiments, the handle assembly 16 supports batteries (not shown) that provide energy to the handle assembly 12 to operate the stapling device 10. Although the stapling device 10 is illustrated as a powered stapling device, it is envisioned that the advantages of this disclosure are suitable for use with manually powered surgical stapling devices as well as robotically controlled stapling devices.

The tool assembly 16 of the stapling device 10 includes a cartridge assembly 30 and an anvil 32. As is known in the art, the cartridge assembly 30 and the anvil 32 are coupled together such that the tool assembly 16 can pivot between an open position and a clamped position. The cartridge assembly 30 includes an exemplary embodiment of the disclosed staple cartridge shown generally as staple cartridge 40.

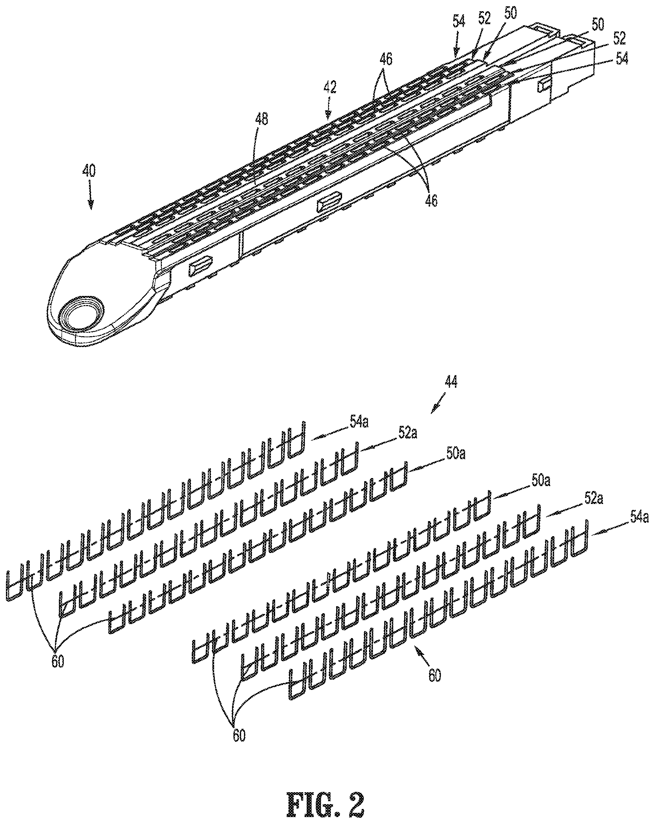

FIG. 2 illustrates the staple cartridge 40 which includes a cartridge body 42 and a plurality of staples 44. The cartridge body 42 defines a plurality of staple pockets 46 and a central knife slot 48 that extends along a midline of the cartridge body 42. As known in the art, the central knife slot 48 facilitates translation of a knife bar (not shown) through the tool assembly 16 to eject staples supported within a staple cartridge and cut tissue clamped between the cartridge assembly 30 and anvil 32. U.S. Patent No. 5,865,361 discloses a known manually powered stapling device including a knife bar that is movable through the tool assembly to eject staples from a staple cartridge and to cut tissue clamped between an anvil and a cartridge assembly of the stapling device. Although the cartridge body 42 is illustrated as being linear, it is envisioned that the cartridge body 42 may have a non-linear configuration or curved along its longitudinal axis.

In aspects of the disclosure, the staple pockets 46 are arranged in rows 50, 52, and 54 that are positioned on each side of the central knife slot 48 in the staple pockets 46 of the cartridge body 42. Each of the staple pockets 46 supports a staple 44 such that the staples 44 are aligned in rows 50a, 52a, and 54a on each side of the central knife slot 48 within the cartridge body 42 of the staple cartridge 40. It is noted that the rows need not be linear but rather may be curved along the longitudinal axis of the cartridge body 42. The inner rows 50a of staples 44 are positioned closest to and on opposite sides of the central knife slot 48. The middle and outer rows 52a and 54a of the staples 44 are positioned outwardly of the central knife slot 48 and of the inner rows 50a of staples 44 on opposite sides of the central knife slot 48. Although the staple cartridge 40 is illustrated to include three rows of staples 44 on each side of the central knife slot 48, it is envisioned that the staple cartridge 40 may include only two rows of staples 44 or four or more rows of staples 44 on each side of the knife slot 48.

In aspects of the disclosure, at least two of the rows of staples are formed of dissimilar metals that have different anodic indexes. In one embodiment, the inner rows 50a of staples 44 are formed of a first metal having a first anodic index and the outer two rows 52a and 54a of staples 44 are formed of a second dissimilar metal having a second anodic index. It is also envisioned that the inner and middle rows 50, 52 of staples 44 can be formed of the same metal having a first anodic index and that the outer row 54 of staples 44 can be formed of a dissimilar metal having a second anodic index different than the first anodic index. In one embodiment, the first metal is magnesium which has an anodic index of -1.75V and the second dissimilar metal is titanium which has an anodic index of -.30V. The dissimilar metals when placed in an electrolyte solution (such as a cellular matrix of tissue, e.g., gastrointestinal tissue) form a galvanic or voltaic cell which uses galvanic potential to drive a voltage within the electrolyte solution, e.g., cellular matrix of tissue.

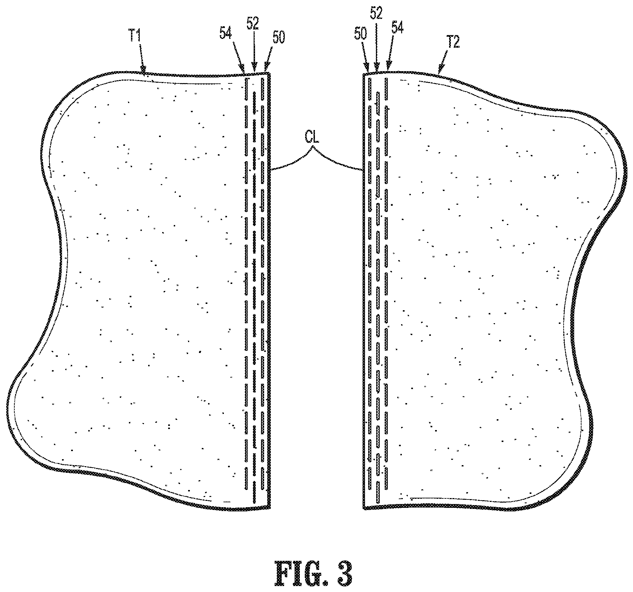

FIG. 3 illustrates dissected tissue sections "T1" and "T2" including staples 44 formed in three rows 50, 52, and 54 along a cut line "CL" in each tissue section "T1" and "T2". In this embodiment, the staples 44 in the inner rows 50 of staples are formed from magnesium and have a first anodic index of -1.75V and the outer two rows 52a and 54a of staples 44 are formed of titanium and have a second anodic index of -.30V. Placing staples 44 formed of one material in the outer row 54 of staples 44 and staples 44 formed of a dissimilar metal in the inner row 50 of staples 44 assists in cell migration towards the cut line "CL". In this embodiment, the staples 44 in the inner row form a cathode and the staples 44 in the outer row 52 or 54 form an anode such that such that a voltage gradient is formed between the anode and the cathode to assist in cell migration toward the cut line "CL". It is envisioned that in some applications, the staples 44 having the lower anodic index may form the outer row or rows 52, 54 of staples 44 and the material having the higher anodic index may form the inner row of staples 44.

It is envisioned that the staple pockets 46 in the cartridge body 44 need not be arranged in rows as illustrated above but rather may be arranged in a variety of different patterns in the cartridge body. In such a cartridge body, the staples nearer to the cut line CL may be formed of a first metal having a first anodic index and the staples spaced further from the cut line "CL" may be formed from a second dissimilar metal having a second anodic index that is different from the first anodic index.

Although it is described in detail herein that all the staples in each of the respective rows are formed of the same material, it is envisioned that only some of the staples in the rows of staples need be formed of a material having a first or second anodic index. For example, every other staple or every third staple 44 in the inner row 50 of staples may be formed from a material, e.g., magnesium, having a first anodic index, and every other or every third staple 44 in one or both of the outer two rows 52a and 54a of staples 44 may be formed of a material, e.g., titanium, having a second anodic index. Other patterns of materials having different anodic indexes are also envisioned to generate local voltages in tissue to drive healing of tissue. Although only magnesium and titanium have been specifically identified herein as materials suitable for use, it is also envisioned that a variety of materials could be used to form some or all of the staples including, e.g., zinc and copper.

It is also envisioned that some or all the staples in the rows of staples can be coupled together by a wire 60 (shown in phantom in FIG. 2) or other conductive material such that the row or portion of the row of staples forms a single conductor. This will prevent local voltages from directing cells away from areas of tissue that have no metal. It is also envisioned that the staples may be used in conjunction with a doped material such as a buttress material to increase healing efficiency as well as prevent local voltages from directing cells away from the areas of tissue with no metal.

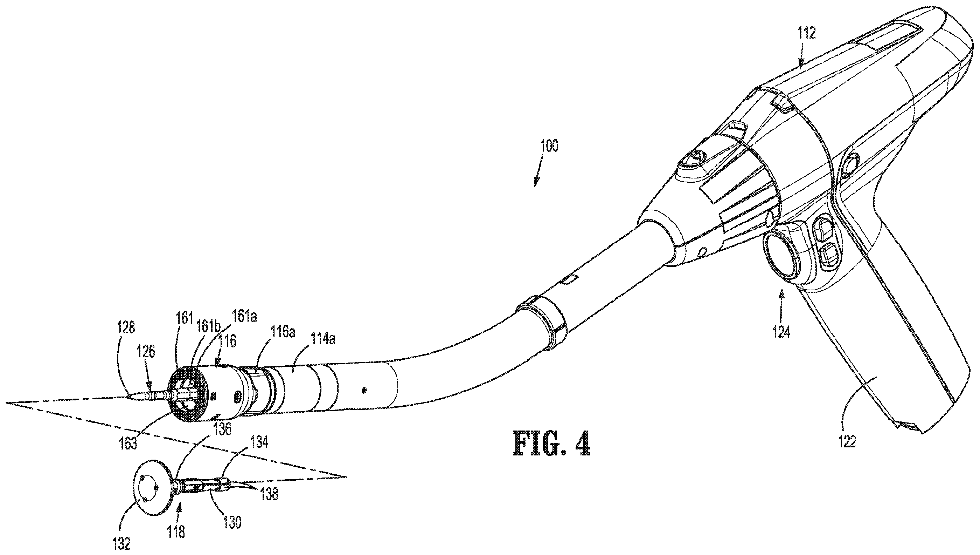

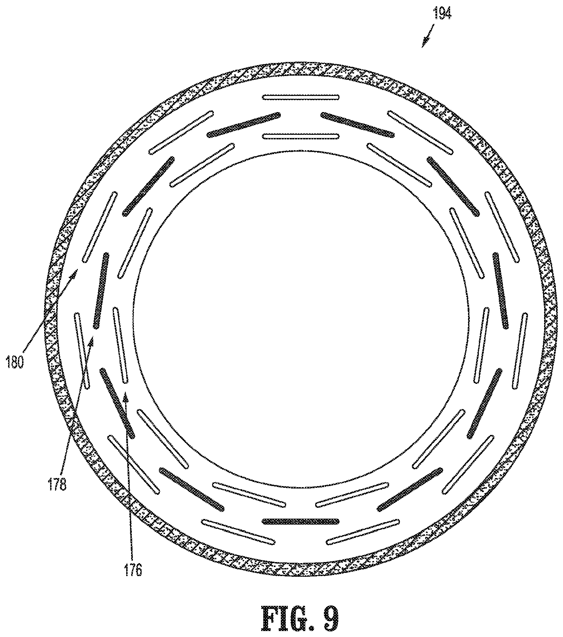

FIGS. 4-9 illustrate an alternate version of the surgical stapling device 10 shown generally as stapling device 100. Stapling device 100 is a circular stapler and includes a handle assembly 112, an elongate body 114, a reload assembly 116, and an anvil assembly 118. The anvil assembly 118 is releasably supported on a distal end of the adaptor assembly 114 and is movable in relation to the reload assembly 116 between spaced and clamped positions as is known in the art. The reload assembly 116 includes a proximal portion 116a that is releasably coupled to a distal portion 114a of the elongate body 114. Alternately, the reload assembly 116 can be fixedly secured to the distal portion 114a of the elongate body 114. In certain aspects of the disclosure, the handle assembly 112 includes a stationary grip 122, and actuation buttons 124 for controlling operation of various functions of the stapling device 100 including approximation of the reload and anvil assemblies 116 and 118, respectively, firing of staples from the reload assembly 116, and cutting or coring of tissue. The elongate body 114 supports an anvil retainer 126 that includes a distal trocar portion 128.

The handle assembly 112 is electrically powered and may include one or more batteries (not shown). The elongate body 114 is in the form of an adaptor assembly that translates power from the handle assembly 112 to the reload and anvil assemblies 116 and 118, respectively. Examples of electrically powered stapling devices can be found in U.S. Patent Nos. 9,055,943, 9,023,014, and U.S. Publication Nos. 2018/0125495, and 2017/0340351. Alternately, it is envisioned that the stapling device 100 could also be a manually powered stapling device such as disclosed in U.S. Patent No. 7,303,106 (the '106 Patent) or a stapling device that is configured for use with a robotic system such as disclosed in U.S. Patent No. 9,962,159 that does not include a handle assembly.

The anvil assembly 118 includes an anvil shaft 130 and an anvil head 132. The anvil shaft 130 includes a proximal portion 134 and a distal portion 136. The proximal portion 134 of the anvil shaft 130 includes a plurality of flexible legs 138 that define a channel (not shown) that receives the anvil retainer 126 to releasably secure the anvil assembly 118 to the anvil retainer 126. For a more detailed description of the releasable connection between the anvil assembly 118 and the anvil retainer 126, see the '106 Patent.

FIGS. 4-6A illustrate the reload assembly 116 which includes a shell housing 160, an annular staple cartridge 161, and staples 150. The staple cartridge 161 is supported on a distal portion of the shell housing 160 and includes an annular cartridge body 161a that defines a central opening and 161b and staple receiving slots or pockets 162 positioned about the central opening 161b. The reload assembly 116 also includes a pusher (not shown) and a cutting blade 163 (FIG. 4) that are supported within the shell housing 160 and are movable between retracted and advanced positions to eject the staples 150 from the staple cartridge 161 and cut tissue clamped between the reload assembly 116 and the anvil assembly 118. In aspects of the disclosure, the cutting blade 163 is coupled to the pusher (not shown) such that movement of the pusher between retracted and advanced positions moves the cutting blade 163 between a retracted position in which the cutting blade 163 is recessed within the cartridge body 161a and an advanced position in which the cutting blade 163 extends distally from the cartridge body 161a.

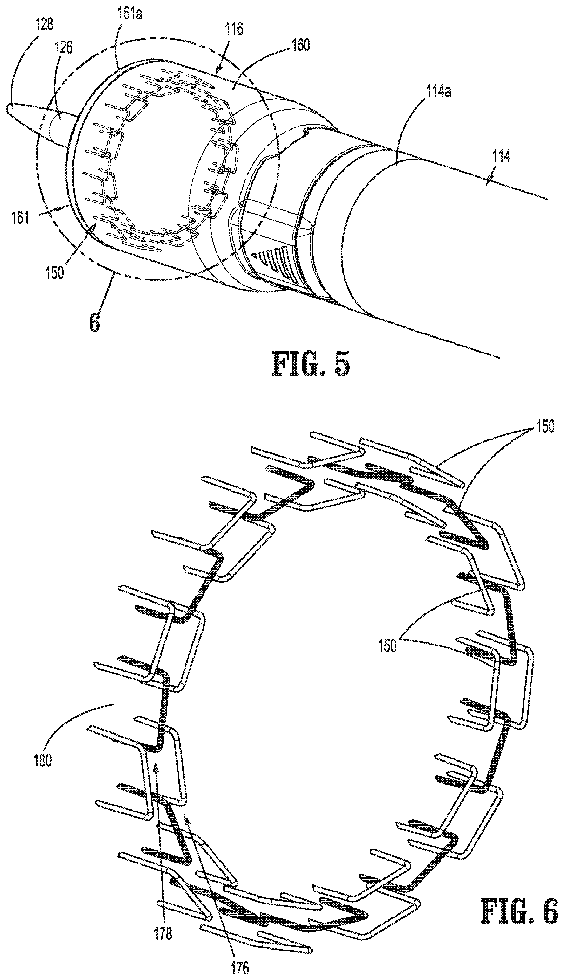

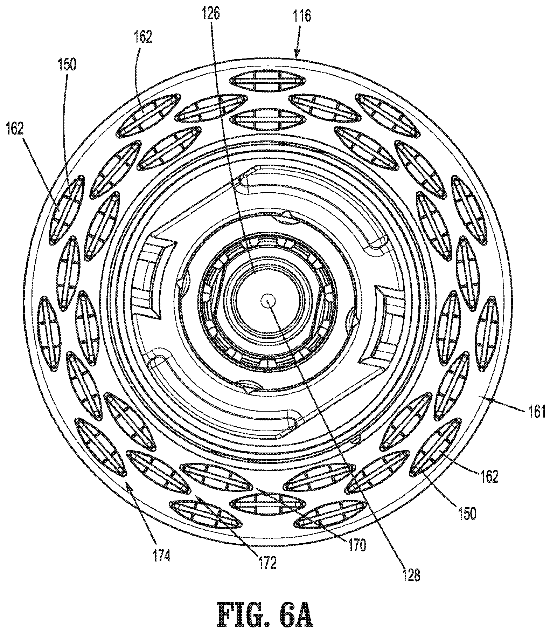

In aspects of the disclosure, the staple receiving pockets 162 are arranged to define rings 170, 172, and 174 (FIG. 6A) that are positioned in annular fashion about central opening defined by the staple cartridge 161. Each of the staple receiving pockets 162 receives one of the staples 150 such that the staples 150 forms rings of staples 150 that are about the central opening of the staple cartridge 161. In aspects of the disclosure, the rings of staples 150 include an inner ring 176, a middle ring 178, and an outer ring 180 (FIG. 6). The inner ring 176 of staples 150 is positioned closest to cutting blade 163 (FIG. 4). The middle and outer rings 178 and 180 of the staples 150 are positioned outwardly of the inner ring 176 of staples150. Although the staple cartridge 161 is illustrated to include three rings of staples 150, it is envisioned that the staple cartridge 161 may include two or more rings of staples 44.

In aspects of the disclosure, at least two of the rings of staples 150 are formed of dissimilar metals that have different anodic indexes. In some aspects of the disclosure, the inner ring of staples 176 is formed of a first metal having a first anodic index and the outer two rings 178 and 180 of staples 150 are formed of a second dissimilar metal having a second anodic index. It is also envisioned that the inner and middle rings 176 and 178 of staples 150 can be formed of the same metal having the first anodic index and that the outer ring 180 of staples 150 can be formed of a dissimilar metal having the second anodic index that is different than the first anodic index. In some aspects of the disclosure, the first metal is magnesium which has an anodic index of -1.75V and the second dissimilar metal is titanium which has an anodic index of -.30V. The dissimilar metals when placed in an electrolyte solution (such as a cellular matrix of tissue, e.g., gastrointestinal tissue) form a galvanic or voltaic cell which uses galvanic potential to drive a voltage within the electrolyte solution, e.g., cellular matrix of tissue.

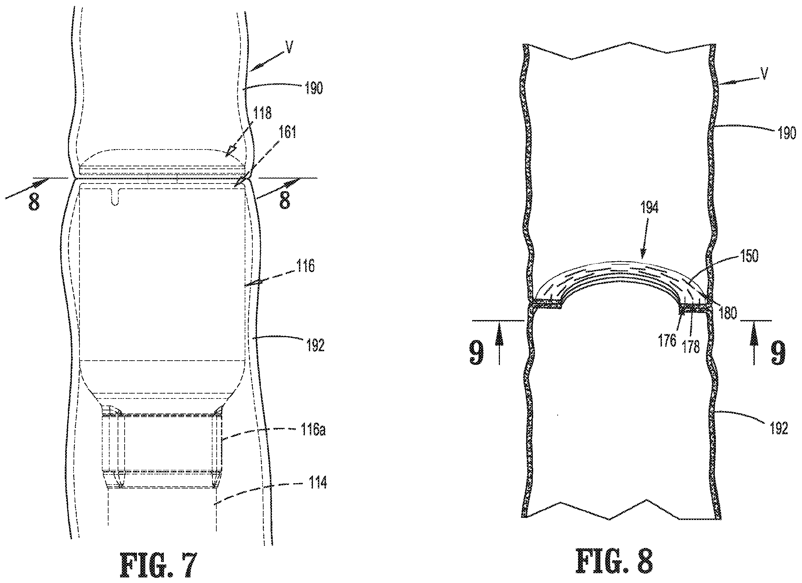

FIGS. 7-9 illustrate the distal portion of the stapling device 100 positioned within a vessel "V", e.g., the colon, after a diseased portion (not shown) of the vessel "V" has been resected during an anastomosis procedure to reconnect first and second tracts 190 and 192, respectively, of the vessel "V". As shown, the distal portion of the stapling device 100 (FIG. 4) is inserted into a lumen "L" defined by the two tracts 190 and 192 of the vessel "V" with the anvil head 132 of the anvil assembly 118 positioned in the first tract 190 and the reload assembly 116 positioned in the second tract 192 of the vessel "V". The anvil head 132 is approximated towards the reload assembly 116 to clamp ends of the two tracts 190 and 192 between the anvil head 132 of the anvil assembly 118 and the staple cartridge 161 of the reload assembly 116. When the anvil head 132 of the anvil assembly 118 and the staple cartridge 161 of the reload assembly 116 are in a clamped position with the ends of the two tracts 190 and 192 of the vessel "V" clamped between the anvil assembly 118 and the staple cartridge 161, the stapling device 100 can be fired to eject the staples 150 from the staple cartridge 161 through both ends of the first and second vessel tracts 190 and 192, respectively, to secure the two tract portions 190 and 192 together and create an anastomotic donut 194.

It is envisioned that the staple pockets 162 in the staple cartridge 161 need not be arranged in rings as illustrated but rather may be arranged in a variety of different patterns in the cartridge body 161a of the staple cartridge 161. In such a staple cartridge, the staples 150 nearer to the cutting blade 163 (FIG. 4) may be formed of the first metal having the first anodic index and the staples 150 spaced further from the cutting blade 163 (FIG. 4) may be formed from the second dissimilar metal having the second anodic index that is different from the first anodic index.

Although it is described in detail herein that all the staples 150 in each of the respective rings 176, 178 and 180 are formed of the same material, it is envisioned that only some of the staples 150 in the rings 176, 178, and/or 180 of staples 150 need be formed of a material having the first or second anodic index. For example, every other staple 150 or every third staple 150 in the inner ring 176 of staples 150 may be formed from a material, e.g., magnesium, having a first anodic index, and every other or every third staple 150 in one or both of the outer two rings 178 and 180 of staples 150 may be formed of a material, e.g., titanium, having a second anodic index. Other patterns of materials having different anodic indexes are also envisioned to generate local voltages in tissue to drive healing of tissue. Although only magnesium and titanium have been specifically identified herein as materials suitable for use, it is also envisioned that a variety of materials could be used to form some or all the staples including, e.g., zinc and copper.

It is also envisioned that some or all the staples in the rows of staples can be coupled together by a wire or other conductive material such that the ring or portion of the ring of staples 150 forms a single conductor. This will prevent local voltages from directing cells away from areas of tissue that have no metal. It is also envisioned that the staples 150 may be used in conjunction with a doped material such as a buttress material to increase healing efficiency as well as prevent local voltages from directing cells away from the areas of tissue with no metal.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described embodiments. Accordingly, the disclosure is not to be limited by what has been particularly shown and described, except as indicated by the appended claims.

## Claims

1. A surgical staple cartridge (40) comprising: an annular cartridge body (42) defining a central opening (48) and staple pockets (46), the staple pockets (46) arranged to define inner and outer rings (176, 180) positioned about the central opening (48), the central opening (48) configured to receive a cutting blade; and a plurality of staples (150), each of the plurality of staple pockets (46) receiving one of the plurality of staples (150), wherein at least some of the plurality of staples (150) received in the inner ring (176) of the staple pockets (46) are formed of a first metal having a first anodic index and at least some of the plurality of staples (150) received in the outer ring (180) of the staple pockets (46) are formed of a second metal having a second anodic index, **characterised in that** the second anodic index is different from the first anodic index, wherein the first anodic index is less than the second anodic index to create a voltaic cell to drive a voltage within tissue to which the plurality of staples (150) are applied to enhance cell migration towards the inner ring (176) closest to the cutting blade.

2. The surgical staple cartridge (40) of claim 1, wherein the first metal is selected from magnesium and zinc, and the second metal is selected from titanium and copper.

3. The surgical staple cartridge (40) of claim 1 or claim 2, wherein the first metal is magnesium and the first anodic index is -1.75V and the second metal is titanium and the second anodic index is -0.30V.

4. The surgical staple cartridge (40) of any one of claims 1 to 3, wherein the staple pockets (46) are arranged to define a middle ring positioned between the inner and outer rings (176, 180), each of the staple pockets (46) of the middle ring receiving one of the staples (150).

5. The surgical staple cartridge (40) of claim 4, wherein at least some of the staples (150) in the middle ring are formed of the second metal.

6. The surgical staple cartridge (40) of claim 4, wherein at least some of the staples (150) in the middle ring are formed of the first metal.

7. A surgical stapling device (40) comprising: an elongate body (42) including an anvil retainer; the staple cartridge (40) according to any of claims 1 to 6; and an anvil assembly supported on the anvil retainer, the anvil assembly being movable in relation to the staple cartridge between advanced and retracted positions to move the stapling device between open and clamped positions.

8. The stapling device of claim 7, further including a handle assembly, the elongate body (42) supported by and extending distally from the handle assembly.

9. The stapling device of any one of claims 7 to 8, wherein the anvil assembly is releasably coupled to the anvil retainer.

10. A surgical staple cartridge (40) according to any one of claims 1 to 6 or a surgical stapling device (10) according to any one of claims 7 to 9, wherein some or all of the staples (150) in rings of staples (150) are coupled together by a wire or other conductive material such that the ring or a portion of the ring of staples (150) forms a single conductor.

## Patentansprüche

1. Chirurgisches Klammermagazin (40) umfassend: einen ringförmigen Magazinkörper (42 150), der eine zentrale Öffnung (48) und Klammertaschen (46) definiert, wobei die Klammertaschen (46) angeordnet sind, um innere und äußere Ringe (176, 180) zu definieren, die um die zentrale Öffnung (48) angeordnet sind, wobei die zentrale Öffnung (48) zum Aufnehmen einer Schneidklinge gestaltet ist; und eine Vielzahl von Klammern (150), wobei jede aus der Vielzahl von Klammertaschen (46) eine aus der Vielzahl von Klammern aufnimmt, wobei wenigstens manche aus der Vielzahl von Klammern (150), die in dem inneren Ring (176) der Klammertaschen (46) aufgenommen sind, aus einem ersten Metall mit einem ersten anodischen Index gebildet sind und wenigstens manche der Vielzahl von Klammern (150), die in dem äußeren Ring (180) der Klammertaschen (46) aufgenommen sind, aus einem zweiten Metall mit einem zweiten anodischen Index gebildet sind, **dadurch gekennzeichnet, dass** der zweite anodische Index von dem ersten anodischen Index verschieden ist, wobei der erste anodische Index kleiner als der zweite anodische Index ist, um eine voltaische Zelle zu erzeugen, um eine Spannung innerhalb von Gewebe zu treiben, in das die Vielzahl von Klammern (150) eingesetzt wird, um Zellmigration in Richtung zu dem inneren Ring (176), der der Schneidklinge am nächsten ist, zu verbessern.

2. Chirurgisches Klammermagazin (40) nach Anspruch 1, wobei das erste Metall ausgewählt ist aus Magnesium und Zink und das zweite Metall ausgewählt ist aus Titan und Kupfer.

3. Chirurgisches Klammermagazin (40) nach Anspruch 1 oder Anspruch 2, wobei das erste Metall Magnesium ist und der erste anodische Index -1,75 V beträgt und das zweite Metall Titan ist und der zweite anodische Index -0,30 V beträgt.

4. Chirurgisches Klammermagazin (40) nach einem der Ansprüche 1 bis 3, wobei die Klammertaschen (46) angeordnet sind, um einen mittleren Ring zu definieren, der zwischen dem inneren und dem äußeren Ring (176, 180) angeordnet ist, wobei jede der Klammertaschen (46) des mittleren Rings eine der Klammern (150) aufnimmt.

5. Chirurgisches Klammermagazin (40) nach Anspruch 4, wobei wenigstens manche der Klammern (150) in dem mittleren Ring aus dem zweiten Metall gebildet sind.

6. Chirurgisches Klammermagazin (40) nach Anspruch 4, wobei wenigstens manche der Klammern (150) in dem mittleren Ring aus dem ersten Metall gebildet sind.

7. Chirurgische Klammervorrichtung (40) umfassend: einen langgestreckten Körper (42), der einen Ambosshalter aufweist; das Klammermagazin (40) nach einem der Ansprüche 1 bis 6; und eine Ambossanordnung, die auf dem Ambosshalter getragen ist, wobei die Ambossanordnung relativ zu dem Klammermagazin zwischen vorgeschobenen und zurückgezogenen Positionen beweglich ist, um die Klammervorrichtung zwischen offenen und geklemmten Positionen zu bewegen.

8. Klammervorrichtung nach Anspruch 7, ferner umfassend eine Griffanordnung, wobei der langgestreckte Körper (42) von der Griffanordnung getragen wird und sich distal davon erstreckt.

9. Klammervorrichtung nach einem der Ansprüche 7 bis 8, wobei die Ambossanordnung lösbar an den Ambosshalter gekoppelt ist.

10. Chirurgisches Klammermagazin (40) nach einem der Ansprüche 1 bis 6 oder chirurgische Klammervorrichtung (10) nach einem der Ansprüche 7 bis 9, wobei manche oder alle der Klammern (150) in Ringen von Klammern (150) durch einen Draht oder ein anderes leitfähiges Material zusammengekoppelt sind, so dass der Ring oder ein Abschnitt des Rings von Klammern (150) einen einzigen Leiter bildet.

## Revendications

1. Cartouche d'agrafes chirurgicales (40) comprenant : un corps de cartouche annulaire (42) définissant une ouverture centrale (48) et des poches d'agrafes (46), les poches d'agrafes (46) étant conçues pour définir des anneaux interne et externe (176, 180) positionnés autour de l'ouverture centrale (48), l'ouverture centrale (48) étant configurée pour recevoir une lame coupante ; et une pluralité d'agrafes (150), chaque poche de la pluralité de poches d'agrafes (46) recevant une agrafe de la pluralité d'agrafes (150), dans lequel au moins certaines agrafes de la pluralité d'agrafes (150) reçues dans l'anneau interne (176) des poches d'agrafes (46) sont formées d'un premier métal ayant un premier indice anodique et au moins certaines agrafes de la pluralité d'agrafes (150) reçues dans l'anneau externe (180) des poches d'agrafes (46) sont formées d'un second métal ayant un second indice anodique, **caractérisée en ce que** le second indice anodique est différent du premier indice anodique, dans lequel le premier indice anodique est inférieur au second indice anodique pour créer une cellule voltaïque pour appliquer une tension dans le tissu auquel la pluralité d'agrafes (150) est appliquée pour améliorer la migration des cellules vers l'anneau interne (176) le plus proche de la lame de coupe.

2. Cartouche d'agrafes chirurgicales (40) selon la revendication 1, dans laquelle le premier métal est choisi parmi le magnésium et le zinc, et le second métal est choisi parmi le titane et le cuivre.

3. Cartouche d'agrafes chirurgicales (40) selon la revendication 1 ou la revendication 2, dans laquelle le premier métal est du magnésium et le premier indice anodique est de -1,75 V et le second métal est du titane et le second indice anodique est de -0,30 V.

4. Cartouche d'agrafes chirurgicales (40) selon l'une quelconque des revendications 1 à 3, dans laquelle les poches d'agrafes (46) sont conçues pour définir un anneau médian positionné entre les anneaux interne et externe (176, 180), chacune des poches d'agrafes (46) de l'anneau médian recevant l'une des agrafes (150).

5. Cartouche d'agrafes chirurgicales (40) selon la revendication 4, dans laquelle au moins certaines des agrafes (150) dans l'anneau médian sont formées du second métal.

6. Cartouche d'agrafes chirurgicales (40) selon la revendication 4, dans laquelle au moins certaines des agrafes (150) dans l'anneau médian sont formées du premier métal.

7. Dispositif d'agrafage chirurgical (40) comprenant : un corps allongé (42) comportant un dispositif de retenue d'enclume ; la cartouche d'agrafes (40) selon l'une quelconque des revendications 1 à 6 ; et un ensemble enclume supporté sur le dispositif de retenue d'enclume, l'ensemble enclume étant mobile par rapport à la cartouche d'agrafes entre des positions avancée et rétractée pour déplacer le dispositif d'agrafage entre des positions ouverte et serrée.

8. Dispositif d'agrafage selon la revendication 7, comportant en outre un ensemble poignée, le corps allongé (42) étant supporté par l'ensemble poignée et s'étendant distalement à partir de celui-ci.

9. Dispositif d'agrafage selon l'une quelconque des revendications 7 à 8, dans lequel l'ensemble enclume est accouplé de manière amovible au dispositif de retenue d'enclume.

10. Cartouche d'agrafes chirurgicales (40) selon l'une quelconque des revendications 1 à 6 ou dispositif d'agrafage chirurgical (10) selon l'une quelconque des revendications 7 à 9, dans lesquels certaines ou toutes les agrafes (150) dans les anneaux d'agrafes (150) sont accouplées ensemble par un fil ou un autre matériau conducteur de telle sorte que l'anneau ou une partie de l'anneau d'agrafes (150) forme un conducteur unique.
